# FASCICULE DE BREVET EUROPEEN

(11) **EP 0 912 654 B1**
(45) Date de publication et mention de la délivrance du brevet: **03.05.2000**
(21) Numéro de dépôt: 97932861.4
(22) Date de dépôt: 08.07.1997
(51) Int. Cl.: C09K 15/08, C07C 7/20

(54) **COMPOSITION EMPECHANT LA POLYMERISATION DE MONOMERES A INSATURATION ETHYLENIQUE, PROCEDE DE PREPARATION ET UTILISATION DE CELLE-CI**
ZUSAMMENSETZUNG UM DIE POLYMERISATION VON ETHYLENISCH UNGESÄTTIGTEN MONOMEREN ZU VERHINDERN, VERFAHREN ZUR HERSTELLUNG UNF VERWENDUNG DERSELBEN
COMPOSITION PREVENTING THE POLYMERISATION OF ETHYLENE UNSATURATED MONOMERS, METHOD OF PREPARATION AND USE THEREOF

(30) Priorité: 12.07.1996 FR 9608771
(43) Date de publication de la demande: 06.05.1999
(73) Titulaire: RHODIA CHIMIE, 92408 Courbevoie Cédex (FR)
(72) Inventeur: LARTIGUE-PEYROU, Françoise, F-69500 Bron (FR)
(74) Mandataire: Le Guen, Gérard
(86) Numéro de dépôt international: FR9701238
(87) Numéro de publication internationale: WO9802500

(56) Documents cités:
- GB-A- 2 295 150
- US-A- 3 390 198
- DATABASE WPI Week 9416 Derwent Publications Ltd., London, GB; AN 94-128841 XP002029267 & JP 06 073 105 A (DAINIPPON) , 3 décembre 1993
- DATABASE WPI Week 9402 Derwent Publications Ltd., London, GB; AN 94-012224 XP002029268 & JP 05 320 095 A (IDEMITSU) , 3 décembre 1993

## Description

L'invention concerne une composition liquide dans la plage allant de la température ambiante à -50° C susceptible d'être utilisée comme inhibiteur de la polymérisation de monomères à insaturation éthylénique, lors de la synthèse de ceux-ci.

Il va de soi que lors de la synthèse de monomères à insaturation éthylénique, une polymérisation desdits monomères est à éviter, d'une part parce qu'elle s'accompagne d'une diminution notable des rendements, d'autre part parce qu'elle provoque simultanément l'encrassement des réacteurs et nécessite un arrêt de la production en raison de problèmes de maintenance.

Or, certains monomères à insaturation éthylénique sont particulièrement sujets à de telles réactions de polymérisation. C'est notamment le cas des mono- et dioléfines et plus particulièrement des dioléfines à doubles liaisons conjuguées, par exemple isoprène et 1,3-butadiène, ainsi que des composés vinyliques.

Lesdites réactions de polymérisation sont spontanées et peuvent avoir lieu en phase liquide, comme en phase gazeuse, en cours de fabrication ou lors de l'utilisation ou de la manipulation desdits monomères.

De façon à empêcher la polymérisation des monomères à insaturation éthylénique, il est connu dans la technique d'ajouter un ou plusieurs inhibiteurs de polymérisation, soit de façon préventive en cours de fabrication desdits monomères, soit encore directement auxdits monomères avant leur utilisation. Ces composés sont généralement des inhibiteurs de polymérisation radicalaire.

Il est également utile dans ce domaine technique de disposer d'un inhibiteur d'urgence capable de stopper la polymérisation desdits monomères dans les procédés classiques de synthèse par polymérisation, dans le cas où survient un problème technique sur l'installation.

Le principal inhibiteur utilisé est le 4-tert-butylcatéchol, lequel est additionné seul ou éventuellement en mélange avec d'autres inhibiteurs.

A ce propos, on pourra se référer à US 2 478 710, US 3 405 189 et JP 19 633/64, lesquels décrivent la stabilisation du butadiène, de l'isoprène, du 1,3-pentadiène et du cyclopentadiène. De même, FR 2 696 171, US 3 390 198 et EP 403 672 illustrent la stabilisation de monomères vinyliques. Plus généralement, on se rapportera à US 2 925 449 et US 4 487 981.

Le 4-tert-butylcatéchol est un composé solide à température ambiante. Son point de fusion est 54° C. Ainsi, de façon à pouvoir être facilement injecté au niveau des rebouilleurs, des réservoirs, des réacteurs, des lignes de stockage et des reflux de distillation des installations industrielles, il doit être solubilisé au préalable soit dans le monomère à stabiliser (à supposer que celui-ci soit liquide), soit dans un solvant organique donné.

Ainsi, le 4-tert-butylcatéchol est commercialisé par Rhône-Poulenc sous forme de composition dans l'eau, dans le méthanol, dans l'isobutanol, dans le toluène et dans le xylène.

Ces compositions présentent cependant l'inconvénient majeur de ne pas être liquides à des températures négatives (inférieures à 0° C). De fait, le 4-tert-butylcatéchol présente un point de cristallisation supérieur à 0° C dans le méthanol, le toluène et le xylène. Par ailleurs, dans des conditions particulières et notamment sous des contraintes vibrationnelles ou mécaniques, une cristallisation partielle du 4-tert-butylcatéchol à partir des compositions correspondantes dans l'eau et l'isobutanol est observée pour une température supérieure à 0° C.

Ainsi, afin d'éviter une cristallisation du 4-tert-butylcatéchol, les solutions commerciales décrites ci-dessus sont préchauffées entre 20 et 60° C et maintenues à ces températures afin d'être injectées sur les procédés de fabrication des monomères cibles, ce qui implique l'utilisation de lignes industrielles thermostatées.

L'invention vise à fournir des compositions à base de 4-tert-butylcatéchol utilisables en tant qu'inhibiteur de polymérisation ne présentant pas les inconvénients des compositions de l'art antérieur, c'est-à-dire liquides à des températures allant de la température ambiante (25° C) jusqu'à environ -50° C. A ces températures en effet, les compositions de l'invention restent sous forme liquide sans qu'il soit possible d'observer la cristallisation, ou la vitrification du 4-tert-butylcatéchol.

Les compositions de l'invention sont non-aqueuses.

Plus précisément, les compositions de l'invention qui sont liquides dans la plage allant de la température ambiante à -50° C comprennent au moins:
a) un ou plusieurs dérivés du catéchol,
b) un solvant aromatique, et
c) un alcool.

De manière préférée, les compositions de l'invention comprennent au moins :
a) un ou plusieurs dérivés du catéchol de formule (I) 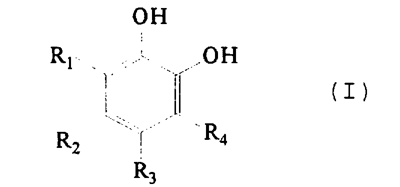 dans laquelle R₁, R₂, R₃ et R₄, identiques ou différents, représentent un atome d'hydrogène, un groupe alkyle ou un groupe alkoxy, étant entendu qu'au moins un substituant parmi R₁, R₂, R₃ et R₄ représente un atome d'hydrogène;
b) un solvant aromatique choisi parmi naphtalène et benzène éventuellement substitués par un ou plusieurs alkyle, et leurs mélanges; et
c) un alcool de formule A-OH dans laquelle A représente alkyle ou cycloalkyle.

Par "alkyle" on entend selon l'invention des chaînes hydrocarbonées linéaires ou ramifiées telles que, par exemple, méthyle, n-butyle, tert-butyle, n-propyle, isopropyle et octyle.

Par "alkoxy" on entend selon l'invention le groupe alkyl-oxy où alkyle est tel que défini précédemment. De préférence, alkoxy désigne isopropoxy.

Des exemples préférés de cycloalkyle sont le cyclopentyle et le cyclohexyle.

Il doit être entendu par ailleurs que dans le cadre de l'invention l'expression "dérivé du catéchol" englobe le catéchol en tant que tel, lequel correspond au composé de formule (I) dans laquelle R₁, R₂, R₃ et R₄ représentent tous un atome d'hydrogène.

Dans la suite, on définit des groupes préférés de dérivés du catéchol, de solvants aromatiques et d'alcools de formule A-OH.

Les compositions préférées de l'invention sont celles comprenant un ou plusieurs dérivés préférés du catéchol comme seuls dérivés du catéchol et/ou comprenant un solvant aromatique préféré et/ou comprenant un alcool préféré de formule A-OH.

Un premier groupe préféré de dérivés du catéchol est celui constitué des composés de formule (I) dans laquelle R₁, R₂, R₃ et R₄, identiques ou différents, sont choisis parmi un atome d'hydrogène, un groupe alkyle en C₁-C₁₂, de préférence en C₁-C₄ et un groupe alkoxy en C₁-C₁₂, de préférence en C₁-C₄.

Un autre groupe de dérivés préférés du catéchol est constitué des composés de formule (I) dans lesquels au moins trois substituants parmi R₁, R₂, R₃ et R₄ représentent un atome d'hydrogène. Parmi ceux-ci, ceux pour lesquels le quatrième substituant R₁, R₂, R₃ ou R₄ est choisi parmi un atome d'hydrogène, un groupe alkyle en C₁-C₁₂, de préférence en C₁-C₄ et un groupe alkoxy en C₁-C₁₂, de préférence en C₁-C₄, sont encore plus avantageux.

Un troisième groupe de composés préférés est celui constitué du catéchol, du 3-méthylcatéchol, du 4-méthylcatéchol, du 3-butyl-5-méthylcatéchol, du 4-tert-butylcatéchol, du 3,5-di-tert-butylcatéchol, du 4,6-di-tert-butylcatéchol, du 4-isopropoxycatéchol, du 3-octyl-5-méthylcatéchol, du 3,6-diisopropylcatéchol et du 3-isopropylcatéchol.

Parmi ces dérivés du catéchol, le 4-tert-butylcatéchol est le plus préféré.

On notera que tous des dérivés du catéchol sont commercialisés ou facilement préparés par l'homme du métier.

Le solvant aromatique est de préférence choisi parmi le naphtalène ou les alkyl-benzènes.

Toutefois, les fractions issues de la distillation fractionnée de coupes pétrolières contenant un ou plusieurs solvants aromatiques sont également appropriées comme solvant.

Lorsque le solvant aromatique est un alkylnaphtalène ou un alkyl-benzène, on préfère que le noyau aromatique soit substitué par un ou plusieurs alkyle en C₁-C₄, de préférence un ou deux alkyle en C₁-C₄.

Comme solvant aromatique particulièrement préféré, on peut citer le naphtalène, le toluène, le benzène, le p-xylène, l'o-xylène, le m-xylène et leur mélange, le toluène étant le solvant aromatique le plus approprié.

Selon l'invention, on peut utiliser avantageusement un mélange des o-, p- et m-xylènes.

Les alcools de formule A-OH particulièrement recommandés dans le cadre de l'invention sont ceux dans lesquels A représente alkyle en C₁-C₁₀ ou cycloalkyle en C₄-C₁₀, notamment l'isopropanol, le méthanol, l'éthanol, le 1-propanol, l'isobutanol, le cyclohexanol, le 1-pentanol, le 1-hydroxy-1,1-diméthylpropane, le 1-octanol et leurs mélanges, étant entendu que l'isopropanol est l'alcool préféré entre tous.

Les proportions massiques idéales de ces constituants (a - le ou les dérivés du catéchol, b- le solvant aromatique et c - l'alcool de formule A-OH) sont facilement déterminées par l'homme du métier, le but étant d'assurer une stabilité maximale du dérivé de catéchol qui ne doit pas se solidifier ou cristalliser dans la plage de température considérée.

Un intérêt particulier sera accordé aux compositions de l'invention constituées :
- de 30 à 60 %,mieux encore de 45 à 55 % en poids de dérivé(s) du catéchol;
- de 20 à 60%, mieux encore de 20 à 30 % en poids dudit solvant aromatique; et
- de 10 à 30 %, mieux encore de 20 à 30 % en poids dudit alcool de formule A-OH.

Le procédé de préparation des compositions de l'invention est quelconque du moment qu'il conduit à une composition homogène.

On peut en effet mélanger dans n'importe quel ordre les constituants des compositions revendiquées, à température ambiante ou à une température supérieure, par exemple comprise entre 50 et 60° C.

Une manière avantageuse de procéder consiste à mettre en oeuvre les étapes de :
(i) solubilisation d'un ou plusieurs dérivés du catéchol dans ledit solvant aromatique;
(ii) addition de l'alcool de formule A-OH au milieu réactionnel obtenu à l'étape (i) et
(iii) éventuellement chauffage du milieu réactionnel résultant de l'étape (ii) à une température comprise entre 50 et 60° C.

Les compositions de l'invention sont des agents inhibiteurs de polymérisation. Ils peuvent donc être utilisés en tant que stabilisants des monomères à insaturation éthylénique les plus susceptibles de se dégrader par polymérisation, soit par addition directe auxdits monomères purs, soit par addition à des solutions desdits monomères.

Toutefois, selon un mode de réalisation préféré, les compositions de l'invention sont utilisées en tant qu'inhibiteur de polymérisation en cours de synthèse desdits monomères. Ceux-ci sont ajoutés au milieu réactionnel à n'importe quelle étape du procédé de synthèse.

Selon un autre de ses aspects, l'invention concerne un procédé destiné à empêcher la polymérisation d'un monomère à insaturation éthylénique lors de la synthèse dudit monomère, comprenant l'addition au milieu réactionnel d'une composition de l'invention, à une étape quelconque dudit procédé de synthèse.

Il n'est pas utile d'ajouter une grande quantité d'inhibiteur de polymérisation. La quantité optimale devant être additionnée dépend de la nature du monomère à insaturation éthylénique à stabiliser.

D'ordinaire, la composition de l'invention est additionnée en une quantité telle que la concentration en dérivés du catéchol est comprise entre 10 et 300 ppm, de préférence entre 50 et 120 ppm.

Les compositions de l'invention sont particulièrement appropriées dans le cas où le monomère à insaturation éthylénique est choisi parmi les monomères vinyliques, le cyclopentadiène et le dicyclopentadiène.

Selon l'invention, l'expression "monomère vinylique" englobe tous les composés présentant au moins un groupe vinyle. Parmi ceux-ci on peut citer le styrène et ses dérivés du type de l'α-méthylstyrène, du vinyltoluène et du divinylbenzène; l'acide acrylique et ses esters tels que l'acrylate de méthyle, l'acrylate d'éthyle et l'acrylate de butyle; les esters de l'acide méthacrylique tels que le méthacrylate de méthyle; la méthylvinylcétone; l'acrylonitrile; l'isoprène; le 2,3-diméthylbuta-1,3-diène; le 1,3-butadiène; le chloroprène; le bromoprène; le 1-chlorobutadiène; le chlorure de vinyle et le 1,3-pentadiène.

Le procédé de l'invention est particulièrement efficace lorsque le monomère à insaturation éthylénique est le 1,3-butadiène ou l'isoprène.

On notera que le procédé de l'invention est également utilisable lors de l'isolement et la purification de mélanges d'hydrocarbures en C₄ issus du vapocraquage de coupes pétrolières et plus particulièrement lorsque ceux-ci contiennent au moins 43 % de 1,3-butadiène.

L'invention sera mieux comprise à la lumière des exemples suivants proposés à titre d'illustration.

Dans tous les exemples les pourcentages sont des pourcentages massiques, et le dérivé du catéchol est le 4-tert-butylcatéchol.

### EXEMPLE I

Dans cet exemple, deux compositions à base de 4-tert-butylcatéchol, de toluène et d'isopropanol ont été préparées par mélange à température ambiante desdits constituants dans les proportions indiquées dans le tableau 1 suivant.

Lesdites compositions ont été étudiées par analyse calorimétrique différentielle sur l'appareil METTLER DSC 30 entre -150 et 100°C de façon à mettre en évidence les transitions solide/liquide. Dans cette plage de température, la cristallisation du 4-tert-butylcatéchol n'a pas été observée.

Les températures de transition vitreuse (T_{g}) mesurées sont indiquées dans le tableau 1 ci-dessous.

**TABLEAU 1**

| Composition | 4-tert-butylcatéchol (%) | toluène | isopropanol | Tg |
|---|---|---|---|---|
| 1 | 50 | 25 | 25 | -98° C |
| 2 | 50 | 30 | 20 | -92° C |

### EXEMPLE II

Dans cet exemple, on a soumis les compositions 3 à 7 du tableau 2 ci-dessous au test suivant de congélation : chacune des compositions est maintenue pendant 8 jours à une température de -20° C. A l'expiration de cette période l'état de la composition est relevé. Les résultats obtenus sont rapportés dans le tableau 2 ci-dessous. Là encore, les compositions de l'invention ont été obtenues par simple mélange des constituants. On notera que le xylène utilisé est un xylène technique constitué d'un mélange de p-xylène, de m-xylène et de o-xylène.

**TABLEAU 2**

| Composition | 4-tert-butylcatéchol | Alkyl-benzène | A-OH | Etat de la composition après 8 jours à -20° C |
|---|---|---|---|---|
| 3 | 30 | toluène 60 | isopropanol 10 | liquide |
| 4 | 30 | toluène 60 | isobutanol 10 | liquide |
| 5 | 30 | xylène 50 | isobutanol 20 | liquide |
| 6 | 40 | xylène 30 | isobutanol 30 | liquide |
| 7 | 30 | xylène 60 | isopropanol 10 | liquide |
| 8 | 50 | xylène 25 | cyclohexanol 25 | liquide |
| 9 | 50 | xylène 30 | octanol-1 20 | liquide |
| 10 | 30 | xylène 40 | éthanol 30 | liquide |
| 11 | 60 | xylène 20 | isobutanol 20 | liquide |
| 12 | 50 | toluène 25 | tertamylalcool 25 | liquide |
| 13 | 50 | toluène 25 | éthanol 25 | liquide |
| 14 | 60 | toluène 27 | isopropanol 13 | liquide |
| 15 | 40 | toluène 30 | cyclohexanol 30 | liquide |

Ainsi qu'il résulte clairement du tableau 2 ci-dessus, les compositions de l'invention sont particulièrement stables à -20° C.

### EXEMPLE III

Dans cet exemple, l'efficacité de la composition 1 de l'exemple 1 à empêcher la polymérisation de l'isoprène à 100° C a été mise en évidence.

Dans un réacteur, on introduit 10 ml d'isoprène, 40 ml de n-heptane et, le cas échéant, la quantité de composition 1 (telle que décrite à l'exemple I) permettant d'atteindre une concentration en 4-tert-butylcatéchol de 30 ppm. Le réacteur est maintenu à 100° C sous une pression d'azote de 4 bars (4.10⁵ Pa) pendant 4 heures. Le milieu réactionnel est alors évaporé sous pression réduite de façon à éliminer les constituants volatils. Le résidu est séché jusqu'à ce que son poids soit constant. Il est constitué du polymère éventuellement formé.

Cette expérience a été réitérée à quatre reprises. Les trois premières fois (témoins 1, 2 et 3) , aucun inhibiteur de polymérisation n'est introduit dans le réacteur. Dans la dernière expérience la composition 1 a été ajoutée à la solution d'isoprène.

Dans chaque cas on a pesé le résidu séché, lequel est indicatif de la masse de polymère formé. Les résultats de ces expériences sont recueillis dans le tableau 3 suivant.

**TABLEAU 3**

| Expérience | Inhibiteur | Concentration en dérivé du catéchol | m (g) du résidu |
|---|---|---|---|
| 1 | néant | - | 133 |
| 2 | néant | - | 130 |
| 3 | néant | - | 137 |
| 4 | composition 1 | 30 ppm | 21 |

Ces résultats indiquent clairement qu'il y a inhibition de la polymérisation par addition de la composition 1 selon l'invention.

## Revendications

1. Composition liquide dans la plage allant de la température ambiante à -50° C comprenant au moins :
a) un ou plusieurs dérivés du catéchol
b) un solvant aromatique, et
c) un alcool.

2. Composition selon la revendication 1, comprenant au moins :
a) un ou plusieurs dérivés du catéchol de formule (I) 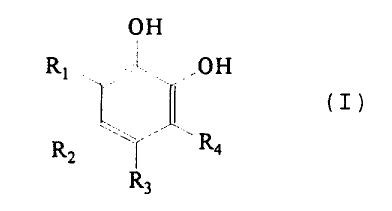 dans laquelle R₁, R₂, R₃ et R₄, identiques ou différents, représentent un atome d'hydrogène, un groupe alkyle ou un groupe alkoxy, étant entendu qu'au moins un substituant parmi R₁, R₂, R₃ et R₄ représente un atome d'hydrogène;
b) un solvant aromatique choisi parmi le naphtalène et les alkyl-benzène dans lesquels le noyau naphténique ou benzénique est substitué par un ou plusieurs alkyle, le benzène et leurs mélanges; et
c) un alcool de formule A-OH dans laquelle A représente alkyle ou cycloalkyle.

3. Composition selon la revendication 2, caractérisée en ce que, dans la formule (I), R₁, R₂, R₃ et R₄, identiques ou différents, sont choisis parmi un atome d'hydrogène, un groupe alkyle en C₁-C₁₂, de préférence en C₁-C₄ et un groupe alkoxy en C₁-C₁₂, de préférence en C₁-C₄.

4. Composition selon l'une quelconque des revendications 2 et 3, caractérisée en ce que dans la formule (I), au moins trois substituants parmi R₁, R₂, R₃ et R₄ représentent un atome d'hydrogène.

5. Composition selon l'une quelconque des revendications précédentes, caractérisée en ce qu'elle comprend un ou plusieurs dérivés du catéchol choisis parmi le catéchol, le 3-méthylcatéchol, le 4-méthylcatéchol, le 3-butyl-5-méthylcatéchol, le 4-tert-butylcatéchol, le 3,5-di-tert-butylcatéchol, le 4,6-di-tert-butylcatéchol, le 4-isopropoxycatéchol, le 3-octyl-5-méthylcatéchol, le 3,6-diisopropylcatéchol et le 3-isopropylcatéchol.

6. Composition selon la revendication 5, caractérisée en ce qu'elle comprend le 4-tert-butylcatéchol.

7. Composition selon l'une quelconque des revendications 2 à 6, caractérisée en ce que le noyau benzénique, respectivement naphténique, desdits solvants aromatiques est substitué par un ou plusieurs alkyle en C₁-C₄.

8. Composition selon l'une quelconque des revendications précédentes, caractérisée en ce que le solvant aromatique est le naphtalène, le toluène, le benzène, le p-xylène, l'o-xylène, le m-xylène ou leurs mélanges, de préférence le toluène.

9. Composition selon l'une quelconque des revendications 2 à 8, caractérisée en ce que l'alcool de formule A-OH est tel que A représente alkyle en C₁-C₁₀ ou cycloalkyle en C₄-C₁₀, l'alcool A-OH étant de préférence choisi parmi l'isopropanol, le méthanol, l'éthanol, le 1-propanol, l'isobutanol, le cyclohexanol, le 1-pentanol, le 1-hydroxy-1,1-diméthylpropane, le 1-octanol et leurs mélanges.

10. Composition selon la revendication 9, caractérisée en ce que l'alcool de formule A-OH est l'isopropanol.

11. Composition selon l'une quelconque des revendications précédentes, caractérisée en ce qu'elle comprend, en pourcentage massique :
- de 30 à 60 %, de préférence de 45 à 55 % d'un ou plusieurs dérivés du catéchol;
- de 20 à 60 %, de préférence de 20 à 30 % dudit solvant aromatique; et
- de 10 à 30 %, de préférence de 20 à 30 % dudit alcool.

12. Composition selon l'une quelconque des revendications précédentes, caractérisée en ce qu'elle est utilisée en tant qu'inhibiteur de la polymérisation de monomères à insaturation éthylénique.

13. Procédé de préparation d'une composition selon l'une quelconque des revendications précédentes, comprenant l'étape consistant à préparer un mélange homogène d'au moins :
a) un ou plusieurs dérivés du catéchol,
b) un solvant aromatique, et
c) un alcool.

14. Procédé selon la revendication 13, caractérisé en ce que ledit mélange homogène est obtenu par mise en oeuvre des étapes suivantes :
(i) solubilisation d'un ou plusieurs dérivés du catéchol dans ledit solvant aromatique;
(ii) addition de l'alcool au milieu réactionnel obtenu à l'étape (i) et
(iii) éventuellement chauffage du milieu réactionnel résultant de l'étape (ii) à une température comprise entre 50 et 60° C.

15. Procédé destiné à empêcher la polymérisation d'un monomère à insaturation éthylénique lors de la synthèse dudit monomère, comprenant l'addition au milieu réactionnel d'une composition selon l'une quelconque des revendications 1 à 12, à une étape quelconque dudit procédé de synthèse.

16. Procédé selon la revendication 15, caractérisé en ce que la quantité de composition additionnée est telle que la concentration en dérivés du catéchol est comprise entre 10 et 300 ppm, de préférence entre 50 et 120 ppm.

17. Procédé selon l'une quelconque des revendications 15 et 16, caractérisé en ce que le monomère à insaturation éthylénique est choisi parmi les monomères vinyliques (tels que le styrène et ses dérivés, l'acide acrylique et ses esters, les esters de l'acide méthacrylique, la méthylvinylcétone, l'acrylonitrile, le 1,3-butadiène, l'isoprène, le 2,3-diméthylbuta-1,3-diène, le chloroprène, le bromoprène, le 1-chlorobutadiène, le chlorure de vinyle et le 1,3-pentadiène), le cyclopentadiène et le dicyclopentadiène.

18. Procédé selon la revendication 17, caractérisé en ce que le monomère à insaturation éthylénique est le 1,3-butadiène ou l'isoprène.

19. Utilisation d'une composition selon l'une quelconque des revendications 1 à 12, comme inhibiteur de polymérisation de monomères à insaturation éthylénique, lors de l'isolement et de la purification de mélanges d'hydrocarbures en C₄ issus du vapocraquage de coupes pétrolières contenant un ou plusieurs monomères à insaturation éthylénique, tels que le 1,3-butadiène ou ses isomères.

20. Utilisation de la composition liquide selon l'une quelconque des revendications 1 à 12 comme inhibiteur d'urgence pendant la synthèse de polymères à partir de monomères à insaturation éthylénique.

## Patentansprüche

1. Im Bereich von Raumtemperatur bis -50°C flüssige Zusammensetzung, die wenigstens folgende Stoffe umfasst:
a) ein oder mehrere Catecholderivate,
b) ein aromatisches Lösungsmittel, und
c) einen Alkohol.

2. Zusammensetzung nach Anspruch 1, die wenigstens folgende Stoffe umfasst:
a) ein oder mehrere Catecholderivate der Formel (I), 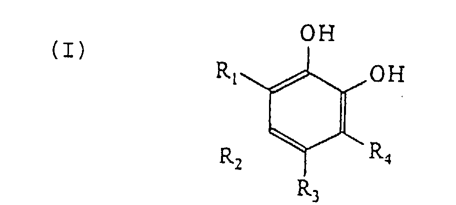 in der R₁, R₂, R₃ und R₄, die gleich oder verschieden sind, ein Wasserstoffatom, eine Alkylgruppe oder eine Alkoxygruppe darstellen, wobei wenigstens einer der Substituenten R₁, R₂, R₃, R₄ ein Wasserstoffatom darstellt;
b) ein aromatisches Lösungsmittel, ausgewählt aus Naphthalin und Alkylbenzolen, in denen der naphthenische Kern oder Benzolkern durch ein oder mehrere Alkyle substituiert ist, Benzol sowie deren Mischungen; und
c) einen Alkohol der Formel A-OH, in der A Alkyl oder Cycloalkyl bedeutet.

3. Zusammensetzung nach Anspruch 2, dadurch gekennzeichnet, dass R₁, R₂, R₃ und R₄, welche gleich oder verschieden sind, aus einem Wasserstoffatom, einer Alkylgruppe mit C₁-C₁₂, vorzugsweise C₁-C₄, und einer Alkoxygruppe mit C₁-C₁₂, vorzugsweise C₁-C₄, ausgewählt sind.

4. Zusammensetzung nach einem der Ansprüche 2 und 3, dadurch gekennzeichnet, dass in der Formel (I) wenigstens drei der Substituenten R₁, R₂, R₃ und R₄ ein Wasserstoffatom darstellen.

5. Zusammensetzung nach einem der vorangehenden Ansprüche, dadurch gekennzeichnet, dass sie ein oder mehrere Catecholderivate aufweist, die aus Catechol, 3-Methylcatechol, 4-Methylcatechol, 3-Butyl-5-Methylcatechol, 4-tert-Butylcatechol, 3,5-di-tert-Butylcatechol, 4,6-di-tert-Butylcatechol, 4-Isopropoxycatechol, 3-Octyl-5-Methylcatechol, 3,6-Diisopropylcatechol und 3-Isopropylcatechol ausgewählt sind.

6. Zusammensetzung nach Anspruch 5, dadurch gekennzeichnet, dass sie 4-tert-Butylcatechol umfasst.

7. Zusammensetzung nach einem der Ansprüche 2 bis 6, dadurch gekennzeichnet, dass der Benzolkern bzw. der naphthenische Kern der aromatischen Lösungsmittel durch ein oder mehrere Alkyle mit C₁-C₄ substituiert ist.

8. Zusammensetzung nach einem der vorangehenden Ansprüche, dadurch gekennzeichnet, dass das aromatische Lösungsmittel Naphthalin, Toluol, Benzol, p-Xylol, o-Xylol, m-Xylol oder eine Mischung derselben, vorzugsweise Toluol, ist.

9. Zusammensetzung nach einem der Ansprüche 2 bis 8, dadurch gekennzeichnet, dass der Alkohol der Formel A-OH dergestalt ist, dass A Alkyl mit C₁-C₁₀ oder Cycloalkyl mit C₄-C₁₀ darstellt, wobei der Alkohol A-OH vorzugsweise aus Isopropanol, Methanol, Ethanol, 1-Propanol, Isobutanol, Cyclohexanol, 1-Pentanol, 1-Hydroxy-1,1-Dimethylpropan, 1-Octanol und deren Mischungen ausgewählt ist.

10. Zusammensetzung nach Anspruch 9, dadurch gekennzeichnet, dass der Alkohol der Formel A-OH Isopropanol ist.

11. Zusammensetzung nach einem der vorangehenden Ansprüche, dadurch gekennzeichnet, dass sie folgende Stoffe in folgenden Masseanteilen aufweist:
- ein oder mehrere Catecholderivate in einem Anteil von 30 bis 60%, vorzugsweise 45 bis 55%;
- das aromatische Lösungsmittel in einem Anteil von 20 bis 60%, vorzugsweise 20 bis 30%; und
- den Alkohol in einem Anteil von 10 bis 30%, vorzugsweise 20 bis 30%.

12. Zusammensetzung nach einem der vorangehenden Ansprüche, dadurch gekennzeichnet, dass sie dazu verwendet wird, die Polymerisation von ethylenisch ungesättigten Monomeren zu hemmen.

13. Verfahren zum Herstellen einer Zusammensetzung nach einem der vorangehenden Ansprüche, mit einem Schritt, der darin besteht, ein homogenes Gemisch herzustellen, das wenigstens folgende Stoffe umfasst:
a) ein oder mehrere Catecholderivate,
b) ein aromatisches Lösungsmittel, und
c) einen Alkohol.

14. Verfahren nach Anspruch 13, dadurch gekennzeichnet, dass das homogene Gemisch durch Ausführung folgender Schritte gewonnen wird:
(i) Solubilisierung eines oder mehrerer Catecholderivate in dem aromatischen Lösungsmittel;
(ii) Beigabe des Alkohols zu dem im Schritt (i) erhaltenen Reaktionsmilieu, und
(iii) eventuell Erwärmung des aus dem Schritt (ii) resultierenden Reaktionsmilieus auf eine Temperatur zwischen 50 und 60°C.

15. Verfahren, das dazu bestimmt ist, bei der Synthese eines ethylenisch ungesättigten Monomers dessen Polymerisation zu verhindern, wobei das Verfahren die Beigabe einer Zusammensetzung nach einem der Ansprüche 1 bis 12 zum Reaktionsmilieu in einem beliebigen Schritt des Syntheseprozesses umfasst.

16. Verfahren nach Anspruch 15, dadurch gekennzeichnet, dass die Zusammensetzung in einer solchen Menge beigegeben wird, dass die Catecholderivatkonzentration zwischen 10 und 300 ppm, vorzugsweise zwischen 50 und 120 ppm, liegt.

17. Verfahren nach einem der Ansprüche 15 und 16, dadurch gekennzeichnet, dass das ethylenisch ungesättigte Monomer aus Vinylmonomeren (wie zum Beispiel Styrol und dessen Derivaten, Akrylsäure und deren Estern, Methacrylsäureester, Methylvinylketon, Acrylnitril, 1,3-Butadien, Isopren, 2,3-Dimethylbuta-1,3-dien, Chloropren, Bromopren, 1-Chlorobutadien, Vinylchlorid und 1,3-Pentadien), Cyclopentadien und Dicyclopentadien ausgewählt ist.

18. Verfahren nach Anspruch 17, dadurch gekennzeichnet, dass das ethylenisch ungesättigte Monomer 1,3-Butadien oder Isopren ist.

19. Verwendung einer Zusammensetzung nach einem der Ansprüche 1 bis 12 zum Hemmen der Polymerisation von ethylenisch ungesättigten Monomeren bei der Isolierung und Reinigung von C₄-Kohlenwasserstoffgemischen, die aus der Dampfzerlegung von Erdölbestandteilen hervorgehen und ein oder mehrere ethylerisch ungesättigte Monomere, zum Beispiel 1,3-Butadien oder dessen Isomere, enthalten.

20. Verwendung der flüssigen Zusammensetzung nach einem der Ansprüche 1 bis 12 als Notfall-Hemmstoff bei der Synthese von Polymeren aus ethylenisch ungesättigten Monomeren.

## Claims

1. Liquid composition ranging from room temperature to -50°C comprising at least:
a) one or more catechol derivatives
b) an aromatic solvent, and
c) an alcohol.

2. Composition according to Claim 1, comprising at least:
a) one or more catechol derivatives of formula (I) 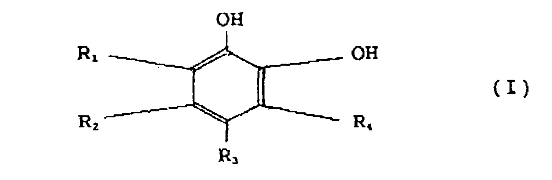 in which R₁, R₂, R₃ and R₄, which are identical or different, represent a hydrogen atom, an alkyl group or an alkoxy group, it being understood that at least one substituent from R₁, R₂, R₃ and R₄ represents a hydrogen atom;
b) an aromatic solvent chosen from naphthalene and alkylbenzenes in which the naphthenic or benzenic core is substituted by one or more alkyls, benzene and their mixtures; and
c) an alcohol of formula A-OH in which A represents alkyl or cycloalkyl.

3. Composition according to Claim 2, characterised in that in the formula (I), R₁, R₂, R₃ and R₄, which are identical or different, are chosen from a hydrogen atom, an alkyl group from C₁-C₁₂, preferably from C₁-C₄ and an alkoxy group from C₁-C₁₂, preferably from C₁-C₄.

4. Composition according to one of Claims 2 and 3, characterised in that in the formula (I), at least three substituents from R₁, R₂, R₃ and R₄ represent a hydrogen atom.

5. Composition according to one of the preceding Claims, characterised in that it comprises one or more catechol derivatives chosen from catechol, 3-methylcatechol, 4-methylcatechol, 3-butyl-5-methylcatechol, 4-tert-butylcatechol, 3,5-di-tert-butylcatechol, 4,6-di-tert-butylcatechol, 4-isopropoxycatechol, 3-octyl-5-methylcatechol, 3,6-diisopropylcatechol and 3-isopropylcatechol.

6. Composition according to Claim 5, characterised in that it comprises 4-tert-butylcatechol.

7. Composition according to one of Claims 2 to 6, characterised in that the benzenic core, respectively naphthenic core, of said aromatic solvents is substituted by one or more alkyls from C₁-C₄.

8. Composition according to one of the preceding Claims, characterised in that the aromatic solvent is naphthalene, toluene, benzene, p-xylene, o-xylene, m-xylene or their mixtures, preferably toluene.

9. Composition according to one of Claims 2 to 8, characterised in that the alcohol of formula A-OH is such that A represents alkyl from C₁-C₁₀ or cycloalkyl from C₄-C₁₀, the alcohol A-OH preferably being chosen from isopropanol, methanol, ethanol, 1-propanol, isobutanol, cyclohexanol, 1-pentanol, 1-hydroxy-1,1-dimethylpropane, 1-octanol and their mixtures.

10. Composition according to Claim 9, characterised in that the alcohol of formula A-OH is isopropanol.

11. Composition according to one of the preceding Claims, characterised in that it comprises, by weight:
- from 30 to 60%, preferably from 45 to 55% of one or more catechol derivatives;
- from 20 to 60%, preferably from 20 to 30% of said aromatic solvent; and
- from 10 to 30%, preferably from 20 to 30% of said alcohol.

12. Composition according to one of the preceding Claims, characterised in that it is used as an inhibitor for the polymerisation of ethylene unsaturated monomers.

13. Method for the preparation of a composition according to one of the preceding Claims, comprising the stage consisting of preparing a homogeneous mixture of at least:
a) one or more catechol derivatives,
b) an aromatic solvent, and
c) an alcohol.

14. Method according to Claim 13, characterised in that said homogeneous mixture is obtained by carrying out the following stages:
(i) dissolving one or more catechol derivatives in said aromatic solvent;
(ii) adding the alcohol to the reaction medium obtained in stage (i) and
(iii) possibly heating the reaction medium resulting from stage (ii) to a temperature of between 50 and 60°C.

15. Method intended to prevent the polymerisation of an ethylene unsaturated monomer at the time of synthesis of said monomer, comprising the addition to the reaction medium of a composition according to one of Claims 1 to 12, at any stage of said method of synthesis.

16. Method according to Claim 15, characterised in that the quantity of composition added is such that the concentration of catechol derivatives is between 10 and 300 ppm, preferably between 50 and 120 ppm.

17. Method according to one of Claims 15 and 16, characterised in that the ethylene unsaturated monomer is chosen from vinyl monomers (such as styrene and its derivatives, acrylic acid and its esters, the esters of methacrylic acid, methylvinylketone, acrylonitrile, 1,3-butadiene, isoprene, 2,3-diroethylbuta-1,3-diene, chloroprene, bromoprene, 1-chlorobutadiene, vinylchloride and 1,3-pentadiene), cyclopentadiene and dicyclopentadiene.

18. Method according to Claim 17, characterised in that the ethylene unsaturated monomer is 1,3-butadiene or isoprene.

19. Use of a composition according to one of Claims 1 to 12, as an inhibitor for the polymerisation of ethylene unsaturated monomers, at the time of separation and purification of mixtures of hydrocarbons from C₄ coming from the vapour-cracking of oil fractions containing one or more ethylene unsaturated monomers, such as 1,3-butadiene or its isomers.

20. Use of the liquid composition according to one of Claims 1 to 12 as an emergency inhibitor during the synthesis of polymers from ethylene unsaturated monomers.
